# EUROPÄISCHE PATENTSCHRIFT

(11) **EP 2 277 599 B1**
(45) Veröffentlichungstag und Bekanntmachung des Hinweises auf die Patenterteilung: **23.01.2019**
(21) Anmeldenummer: 10177136.8
(22) Anmeldetag: 19.03.2007
(51) Int. Cl.: A61Q 5/08, A61K 8/33, A61K 8/35, A61K 8/46, A61K 8/49, D06L 4/30, D06P 5/13

(54) **Reduktiver Farbabzug**
Reductive colour removal
Décoloration réductive

(30) Priorität: 21.03.2006 DE 102006013260; 11.05.2006 DE 102006022225
(43) Veröffentlichungstag der Anmeldung: 26.01.2011
(62) Teilanmeldung aus: 07711979.0
(73) Patentinhaber: Henkel AG & Co. KGaA, 40589 Düsseldorf (DE)
(72) Erfinder: Müller, Burkhard, 21075, Hamburg (DE); Neubueser, Inge, 22587, Hamburg (DE); Groß, Wibke, 41836, Hückelhoven (DE)

(56) Entgegenhaltungen:
- EP-A2- 0 150 405
- DE-C1- 19 647 494
- FR-A1- 2 814 948
- FR-A1- 2 829 764
- US-A1- 2004 034 944

## Beschreibung

Die Erfindung betrifft Zusammensetzungen zum reduktiven Farbabzug keratinhaltiger Fasern, welche neben organischen Sulfinsäurederivaten mindestens eine organische Verbindung, ausgewählt aus Aldehyden und Ketonen, enthalten. Gleichfalls ist ein Verfahren zur Entfärbung gefärbter Substrate, sowie die Verwendung besagter Mittel zum Farbabzug Gegenstand der Erfindung.

Beim Färben wird der Farbstoff auf das Substrat durch Adsorption an die Oberfläche, durch Eindiffundieren, durch Bildung auf und/oder in dem Substrat bzw. durch chemische Bindung übertragen. Für das Färben, beispielsweise von Papier, Textilien oder keratinhaltigen Fasern, kommen im allgemeinen entweder direktziehende Farbstoffe oder Oxidationsfarbstoffe in Frage. Oxidationsfarbstoffe entstehen durch oxidative Kupplung einer oder mehrerer Entwicklerkomponenten untereinander oder mit einer oder mehreren Kupplerkomponenten. Kuppler- und Entwicklerkomponenten werden auch als Oxidationsfarbstoffvorprodukte bezeichnet. Die oxidative Kupplung findet bevorzugt während der Färbevorgangs statt, damit die Farbstoffvorprodukte in das Substrat hinein diffundieren können und der Farbstoff sich in dem Substrat bildet. Durch die Größe des entstandenen Farbstoffmoleküls wird ein Auswaschen aus dem Substrat erschwert.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 4,5-Diamino-1-(2-hydroxyethyl)pyrazol.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Unter direktziehenden Farbstoffen werden im allgemeinen Farbstoffe verstanden, die bereits vor Beginn des Färbens vorgebildet sind und auf das Substrat aufziehen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Ein wichtiges technisches Gebiet stellt neben dem Färben das Abziehen von Farbstoffen dar. Darunter versteht man im Allgemeinen das Entfernen von Färbungen oder Bedruckungen durch Auswaschen, chemische Veränderung oder Zerstörung des Farbstoffes. Die oxidative oder die reduktive Entfärbung gefärbter Materialien findet insbesondere bei der Entfärbung von Textilien oder Haaren Anwendung.

Eine oxidative Entfärbung führt meist zu guten Ergebnissen. Jedoch kann durch die starke Oxidationswirkung des zur Entfärbung benutzten Oxidationsmittels die Struktur des Substrats chemisch verändert werden. Dies geht mit einer unerwünschten physikalischen Änderung des Substrats einher. Textilien oder Haare können beispielsweise spröde werden oder insbesondere bei mehrmaliger Entfärbung gar brechen. Der visuelle Eindruck, die Haptik aber auch die Haltbarkeit des Substrats werden dadurch negativ beeinflusst.

Weniger Einfluss auf die Substratstruktur, insbesondere auf die Struktur keratinhaltiger Fasern, nehmen reduktive Entfärbemittel. Die reduktiven Entfärbemittel entfärben kaum die Naturhaarfarbe, sondern wirken lediglich auf die durch Färbung aufgetragenen Farbstoffe reduktiv ein. Es tritt somit kaum eine Aufhellung des Haars auf. Allerdings bedürfen die reduktiven Entfärbemittel einer Verbesserung der Entfärbeleistung.

Überwiegend nutzt man reduktiv wirkende Schwefelverbindungen zur Entfärbung. Bekanntermaßen eignen sich Dithionite oder Derivate der 1-Hydroxymethylsulfinsäure bzw. der 1-Aminomethylsulfinsäure als Reduktionsmittel.

Aus der Druckschrift US-A-3 892 845 sind dem Fachmann reduktive Farbabzugsmittel bekannt, mit deren Hilfe sich gefärbte keratinhaltige Fasern entfärben lassen. Als Reduktionsmittel ist 1-Hydroxymethylsulfinsäure oder ein Salz davon in den Entfärbemitteln enthalten. Gemäß DE-OS-1 617 829 lässt sich durch Zugabe von 1-Hydroxymethylsulfinsäure zu oxidativen Entfärbemitteln, enthaltend Wasserstoffperoxid und Persulfate, deren Bleichwirkung verstärken.

Laut Offenbarung der EP-A-1 079 018 eignen sich Aminoalkansulfinate der Formel R¹_{3-z}N (CR²R³-SO₂-M)_{z} (R², R³ = Wasserstoff oder (C₁ bis C₄)-Alkyl) zur teilweisen Entfärbung von mit Küpenfarbstoffen oder Schwefelfarbstoffen gefärbten oder bedruckten Textilien.

Gemäß WO-A1-99/18067 eignen sich 1-Hydroxyalkylsulfinsäuren bzw. 1-Aminoalkylsulfinsäuren, welche eine Carboxygruppe, eine Sulfonsäuregruppe, eine Acylgruppe, eine Aminocarbonylgruppe oder eine Alkoxycarbonylgruppe tragen, zur Entfärbung gefärbter Substrate. Besagte Derivate besitzen eine vergleichbare oder bessere Entfärbekraft wie die 1-Hydroxymethylsulfinsäure (*vide supra*). In der WO-A1-02/30369 wird die Entdeckung beschrieben, dass sich mit den SulfinsäureDerivaten der WO-A1-99/18067 auch keratinhaltige Fasern, wie beispielsweise Haare, entfärben lassen. Weitere zur Entfärbung keratinhaltiger Fasern geeignete Sulfinsäurederivate finden sich in den Druckschriften WO-A1-03/026597 und WO-A1-03/41668.

In US 2004/0034944 A1 wird offenbart, dass zur Entfärbung von gefärbten keratinischen Fasern eine Kombination aus Hydroxyalkylsulfinsäuren und α-Oxocarbonsäuren verwendet werden kann. Das Dokument DE 196 47 494 C1 offenbart den Einsatz von Ascorbinsäure als Reduktionsmittel. Weiterhin beschreibt Dokument EP 0 150 405 A2 ein Verfahren zum Entfärben von Textilien, bei welchem eine Mischung aus einer aromatischen N-alkylsulfinsäure und einer Hydroxyalkylsulfinsäure verwendet wird. Schließlich beschreiben die Dokumente FR 2 829 764 A1 sowie FR 2 814 948 A1 die Enfärbung von keratinischen Fasern unter Verwendung von bestimmten Sulfinsäurederivaten. Den zuvor genannten Dokumenten lässt sich jedoch keine spezifische Kombination von bestimmten Sulfinsäurederivaten und Aldehyden zur Entfärbung von keratinischen Fasern entnehmen.

Substrate, die mit den Sulfinsäurederivaten des Standes der Technik reduktiv entfärbt wurden, erfahren einige Zeit nach dem Farbabzug eine unerwünschte Nachdunkelung.

Aufgabe der vorliegenden Erfindung ist es, reduktive Entfärbemittel bereitzustellen, die das Substrat dauerhaft ohne Nachdunkelung entfärben. Die Substratstruktur soll dabei geschont werden. Ferner sollten die in den Entfärbemitteln eingesetzten Reduktionsmittel für eine kosmetische Verwendung physiologisch verträglich und toxikologisch unbedenklich sein.

Überraschenderweise wird die Aufgabe durch Mittel gelöst, die eine Kombination mindestens eines der erfindungsgemäßen organischen Sulfinsäurederivate mit mindestens einer ausgewählten Carbonylverbidnung enthalten. Die Entfärbung gefärbter Substrate in Form von keratinhaltigen Fasern, insbesondere menschliches Haar, gelingt sehr gut ohne dass sich nach dem Entfärbevorgang eine Nachdunkelung in dem hohen Maße einstellt, wie sie durch die Verwendung der Sulfinsäurederivate allein hervorgerufen wird. Die erfindungsgemäßen Mittel eignen sich besonders zur faserschonenden Entfärbung keratinhaltiger Fasern.

Unter keratinhaltigen Fasern sind beispielsweise Wolle, Pelze, Federn und insbesondere menschliche Haare zu verstehen.

Ein erster Gegenstand der Erfindung sind daher Mittel zum reduktiven Farbabzug keratinhaltiger Fasern, die in einem Träger mindestens ein Sulfinsäurederivat der Formel (I) 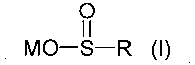 worin
- M: steht für ein Wasserstoffatom oder ein Äquivalent eines ein- oder mehrwertigen Kations,
- R: für einen Rest gemäß einer der Formeln (II) bis (III) steht, 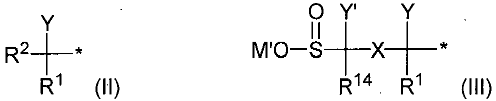 worin bedeuten
Y und Y' unabhängig voneinander eine Hydroxygruppe, eine -NH₂ Gruppe oder eine Gruppe -NR³R⁴, wobei R³ und R⁴ unabhängig voneinander für eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine Arylgruppe oder eine Aryl-(C₁ bis C₆)-alkylgruppe stehen,
M' unabhängig von M die unter M aufgeführten Merkmale,
X eine direkte Bindung oder einen organischen Rest mit zwei freien Valenzen,
R¹ und R¹⁴ unabhängig voneinander ein Wasserstoffatom, eine (C₁ bis C₆)-Alkylgruppe oder eine Carboxyalkylgruppe -(CH₂)ₘ-COOM^{II}, in der M" für ein Wasserstoffatom oder für ein Äquivalent eines ein- oder mehrwertigen Kations steht und m die Zahl 0, 1 oder 2 bedeutet,
R² ein Wasserstoffatom, eine (C₁ bis C₆)-Alkylgruppe, einen Carboxyalkylrest -(CH₂)ₙ-COOM^{III} mit
M^{III} = Wasserstoffatom oder ein Äquivalent eines ein- oder mehrwertigen Kations und n eine ganze Zahl 0, 1, 2, 3, 4, 5 oder 6,
einen (C₁ bis C₆)-Alkyloxycarbonylrest, eine Sulfonsäuregruppe, eine Carbamoylgruppe, eine N,N-Di[(C₁ bis C₆)-alkyl]carbamoylgruppe, eine N,N,N-Tri[(Ci bis C₆)-alkyl]ammonium-(C₁ bis C₆)-alkylgruppe eine Carboxy-(C₂ bis C₆)-alkenylgruppe, eine Cyano-(Ci bis C₆)-alkylgruppe oder eine (C₁ bis C₆)-Alkoxycarbonyl-(C₁ bis C₆)-alkylgruppe, oder
R² zusammen mit R¹ und dem Restmolekül einen aliphatischen 5-, 6-, oder 7-gliedrigen Ring bildet, welcher mindestens ein kationisches, quaterniertes Stickstoffatom als Heteroatom enthält, wobei die kationische Ladung gegebenenfalls durch ein Äquivalent eines ein- oder mehrwertigen Anions kompensiert wird, oder einen Rest gemäß Formel (IV) 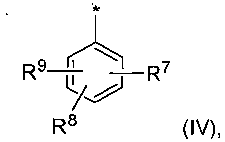 worin
R⁷ eine Carboxygruppe, eine Sulfonsäuregruppe, eine (C₁ bis C₆)-Alkylgruppe, eine (C₂ bis C₆)-Alkenylgruppe, eine Aryl(C₁ bis C₆)-alkylgruppe oder eine (C₁ bis C₆)-Hydroxyalkylgruppe bedeutet,
R⁸ und R⁹ unabhängig voneinander ein Wasserstoffatom, eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine (C₁ bis C₆)-Alkoxygruppe, eine Hydroxygruppe, eine Aminogruppe, eine Carboxygruppe, eine Nitrogruppe, eine Cyanogruppe oder ein Halogenatom bedeuten, mit der Maßgabe, dass in Formel (II) R¹ und R² nicht gleichzeitig für ein Wasserstoffatom stehen
in Kombination mit mindestens einer Verbindung, ausgewählt aus den Aldehyden, wobei die Verbindung aus den Aldehyden ausgewählt wird aus mindestens einem Vertreter aus der Gruppe, die gebildet wird, aus cyclischen, linearen oder verzweigten aliphatischen Aldehyden und wobei die cyclischen, linearen oder verzweigten Aldehyde ausgewählt werden aus mindestens einem Vertreter aus der Gruppe Formaldehyd, Acetaldehyd, Glyoxal, Propionaldehyd, Butanal, Pentanal, Isopentanal, Hexanal, Cyclohexanal, Heptanal, Octanal, Malondialdehyd, Glutaraldehyd, 2-Methylpentanal, 2-Ethylhexanal, 3,5,5-Trimethylhexanal, 2-Ethylbutyraldehyd, 2-Methylbutyraldehyd, Isobutyraldehyd, 3-Phenylpropanal, 3-(4-Methylphenyl)propanal, 3-(4-Methoxyphenyl)propanal, 3-(2-Methoxyphenyl)propanal, 2-Butenal, Acrolein, 3-Methyl-2-butenal, 3,7-Dimethyl-2,6-octadienal, 2,4-Pentadienal, 3,7-Dimethyl-6-octenal, 2,4-Dimethyl-3-cyclohexencarboxaldehyd und 2,6-Nonadienal.
Falls die Verbindungen gemäß Formel (I) mindestens ein Chiralitätszentum enthalten, sind selbstverständlich alle Stereoisomere allein sowie deren Mischungen, insbesondere deren Racemate, erfindungsgemäß.
Die Verbindungen gemäß Formel (I) können neben der Form als freie Säure oder als dessen Salz auch als inneres Salz vorliegen, insbesondere dann, wenn neben der Sulfinat-Gruppe der Formel (I) zusätzlich ein kationischer Substituent (siehe Definition R² und R⁷) im Molekül enthalten ist.

Wenn die Verbindung der Formel (I) als Säure vorliegt, bedeuten die Reste M, M', M^{II} und/oder M^{III} ein Wasserstoffatom. Die Fragmente MO- der Formel (I), M'O- der Formel (III) und M^{II}O-bzw. M^{III}O- gemäß R¹ bzw. R² aus Formel (II) bilden in diesem Fall eine Hydroxygruppe. Wenn die Sulfinsäure der Formel (I) als Salz vorliegt, steht mindestens einer der Reste M, M', M^{II} oder M^{III} für ein Äquivalent eines ein- oder mehrwertigen Kations. Das ein oder mehrwertige Kation M^{z+} mit einer Ladungszahl z von eins oder höher dient lediglich aus Gründen der Elektroneutralität zur Kompensation der einfach negativen Ladung des bei Salzbildung 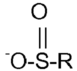 vorliegenden Sulfinat-Fragments aus Formel (I) bzw. mutatis mutandis aus Formel (III), bzw. der Carboxylat-Fragmente der Reste aus R¹ bzw. R² der Formel (II). Das dafür zu verwendende Äquivalent des entsprechenden Kations beträgt 1/z. Das Fragment MO- der Formel (I) bzw. das Fragment M'O- der Formel (III) stehen im Fall der Salzbildung für die Gruppe: 1/z (M^{z+})⁻O- bzw. die Gruppe: 1/z (M'^{z+})⁻O-. Gleiches gilt mutatis mutandis für M^{II} und M^{III} .

Als entsprechende ein- oder mehrwertige Kationen kommen prinzipiell alle Kationen in Frage, die keine Redox-Reaktion mit dem übrigen Sulfinat-Fragment der Formel (I) eingehen. Insbesondere sind dies Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (I) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M, M', M^{II} und M^{III} stehen bevorzugt unabhängig voneinander für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Das Äquivalent des gegebenenfalls vorhandenen ein- oder mehrwertigen Anions gemäß Formel (I) wird, analog zur Definition der Kationäquivalente, zur Wahrung der Elektroneutralität durch Formulierung eines stöchiometrischen Koeffizienten kleiner 1 vor der Bezeichnung des Anions beschrieben. Die besagten Anionen werden im Weiteren definitionsgemäß mit An⁻ symbolisiert. Sie werden bevorzugt ausgewählt aus Halogenid, ½ Sulfat, Hydrogensulfat, ½ Carbonat, Hydrogencarbonat, 1/3 Phosphat, ½ Hydrogenphosphat, Dihydrogenphosphat oder p-Toluolsulfonat. Besonders bevorzugt steht das Anion für Chlorid, Bromid, p-Toluolsulfonat oder Hydrogensulfat.

Erfindungsgemäß sind weiterhin solche Sulfinsäurederivate gemäß Formel (I) bevorzugt, in denen die Reste Y der Formel (II) bzw. Formel (III) und Y' der Formel (III) unabhängig voneinander eine Hydroxygruppe oder eine Gruppe -NH₂ bedeuten.

Sulfinsäurederivate, in denen R¹ für ein Wasserstoffatom oder eine Methylgruppe steht, sind erfindungsgemäß bevorzugt.

Steht der Rest R der Formel (I) für einen Rest der oben genannten Formel (II), dann ergibt sich als bevorzugte erste Ausführungsform aus der Formel (I) das erfindungsgemäße Sulfinsäurederivat der Formel (la), 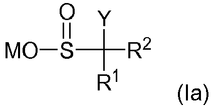 worin M, Y, R¹ und R² die unter den Formeln (I) und (II) definierte Bedeutung haben. Die zuvor erwähnten bevorzugten Definitionen der Reste M, Y und R¹ gelten auch hier, allein oder gemeinsam auf Formel (la) angewendet, als bevorzugt.

Es ist erfindungsgemäß, wenn gemäß einer bevorzugten Ausführungsform der Erfindung der Rest R² gemäß Formel (II) bzw. Formel (la) für einen aliphatischen oder aromatischen Heterozyklus steht, welcher gegebenenfalls substituiert sein kann. Falls die oben genannten ausgewählten aliphatischen oder aromatischen Heterozyklen dieser Ausführungsform substituiert sind, dann sind diese bevorzugt mit mindestens einem Rest aus (C₁ bis C₆)-Alkyl, (C₂ bis C₆)-Alkenyl, (C₁ bis C₆)-Hydroxyalkyl, Aryl-(C₁ bis C₆)-alkyl, Hydroxy, (C₁ bis C₆)-Alkoxy, Amino, Di(C₁ bis C₆)alkylamino, Nitro, Halogen, Carbamoyl, Sulfonamido, Cyano oder Carboxamido, substituiert.

Im Rahmen dieser Ausführungsform ist es wiederum bevorzugt, die aliphatischen oder aromatischen Heterozyklen des Rests R² auszuwählen aus Thienyl, Furyl, Pyrrolyl, Imidazolyl, Thiazolyl, Oxazolyl, Pyrazolyl, Pyridyl, Pyrimidinyl, Pyrazyl, Pyridazyl, Benzimidazolyl, Benzothiazolyl, Benzoxazolyl, Indolyl, Chinolinyl, Chinoxalinyl oder Chinazolinyl, welche gegebenenfalls substituiert sein können, bevorzugt mit den oben genannten Substituenten, besonders bevorzugt mit mindestens einem Rest aus (C₁ bis C₆)-Alkyl, (C₁ bis C₆)-Hydroxyalkyl, Hydroxy, Amino, (C₁ bis C₆)-Alkoxy, Carboxy, Halogenatom, Nitrogruppe oder Sulfonsäurerest.

Dabei sind besonders bevorzugte Sulfinsäurederivate dieser Ausführungsform Verbindungen der nachfolgenden Formeln (la-1) bis (la-4) 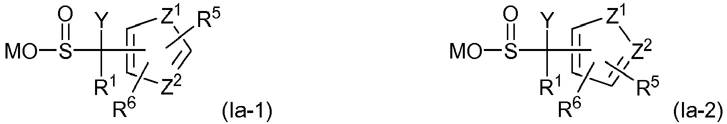 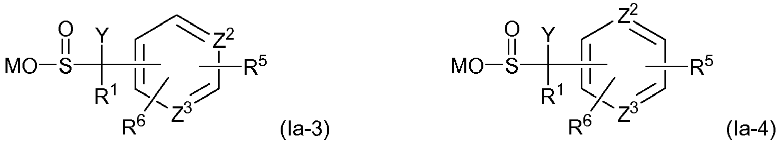 worin
- M, Y und R¹: die unter Formel (I) definierten Bedeutungen haben,
- Z¹: für ein Sauerstoffatom, ein Schwefelatom oder eine Gruppe N-R^{IV} steht, worin R^{IV} ein Wasserstoffatom, eine (C₁ bis C₆)-Alkylgruppe, eine (C₂ bis C₆)-Alkenylgruppe, eine (C₁ bis C₆)-Hydroxyalkylgruppe oder eine Aryl-(C₁ bis C₆)-alkylgruppe bedeutet,
- Z² und Z³: unabhängig voneinander für eine Gruppe CH oder für ein Stickstoffatom stehen,
- R⁵ und R⁶: unabhängig voneinander stehen für ein Wasserstoffatom, eine (C₁ bis C₆)-Alkylgruppe, eine (C₂ bis C₆)-Alkenylgruppe, eine (C₁ bis C₆)-Hydroxyalkylgruppe, eine Hydroxygruppe, eine (C₁ bis C₆)-Alkoxygruppe, eine Aminogruppe, eine Di(C₁ bis C₆)alkylaminogruppe, eine Nitrogruppe, eine Halogenatom, eine Carbamoylgruppe, eine Sulfonamidogruppe, eine Cyanogruppe oder eine Carboxamidogruppe oder gemeinsam eine Benzanellierung bilden, welche wiederum substituiert sein kann.

Die Gruppe N-R^{IV} aus Rest Z¹ bildet im Ring des Heterozyklus eine Azandiylgruppe -NR^{IV}-. Die Gruppe CH der Reste Z² bzw. Z³ bildet im Ring des Heterozyklus eine Methanylylidengruppe =CH-, die selbstverständlich auch mit einem der Reste R⁵ bzw. R⁶ substituiert sein kann.

Wenn in den Formeln (la-1) bzw. (la-2) Z¹ für ein Sauerstoffatom, Z² für eine Gruppe CH und Y für eine Hydroxygruppe steht, so ist es erfindungsgemäß bevorzugt, wenn in den Formeln (la-1) bzw. (la-2) R⁵ und R⁶ nicht gleichzeitig für ein Wasserstoffatom stehen.

Die zuvor erwähnten bevorzugten Definitionen der Reste M, Y und R¹ gelten auch hier, allein oder gemeinsam, auf Formeln (la-1) bis (la-4) angewendet, als bevorzugt.

Die Benzanellierung aus den Resten R⁵ und R⁶ der Formel (la-1) bis (la-4) ist, wenn sie substituiert ist, bevorzugt mit mindestens einem Rest aus (C₁ bis C₆)-Alkyl, (C₂ bis C₆)-Alkenyl, (C₁ bis C₆)-Hydroxyalkyl, Aryl-(C₁ bis C₆)-alkyl, Hydroxy, (C₁ bis C₆)-Alkoxy, Amino, Di(C₁ bis C₆)alkylamino, Nitro, Halogen, Carbamoyl, Sulfonamido, Cyano oder Carboxamido substituiert. R⁵ und R⁶ gemäß Formeln (la-1) bis (la-4) stehen besonders bevorzugt für ein Wasserstoffatom, eine Hydroxygruppe, eine (C₁ bis C₆)-Alkylgruppe, eine (C₁ bis C₆)-Alkoxygruppe, eine (C₁ bis C₆)-Hydroxyalkylgruppe, Halogenatom oder Nitrogruppe.

Wenn die Reste R¹ und R² gemäß Formel (II) im Rahmen der ersten Ausführungsform gemeinsam mit dem Restmolekül einen aliphatischen 5-, 6-, oder 7-gliederigen Ring bilden, welcher mindestens ein kationisches, quaterniertes Stickstoffatom als Heteroatom enthält wobei die kationische Ladung gegebenenfalls durch die ein Äquivalent eines ein oder mehrwertigen Anions kompensiert wird, sind folgende Verbindungen gemäß Formeln (la-5) und (la-6) bevorzugt, 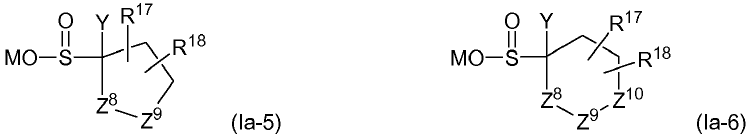 worin
- Y: die unter Formel (I) beschriebenen Definitionen bedeutet,
- M: die unter Formel (I) beschriebenen Definitionen oder eine negative Ladung bedeutet,
- Z⁸, Z⁹ und Z¹⁰: einer dieser Reste eine Azoniumdiyl-Gruppe N⁺R^{V}R^{VI} bedeutet
mit R^{V} und R^{VI} stehen unabhängig voneinander für eine (C₁ bis C₆)-Alkylgruppe, eine (C₂ bis C₆)-Alkenylgruppe, eine (C₁ bis C₆)-Hydroxyalkylgruppe oder eine Aryl-(C₁ bis C₆)-alkylgruppe,
und die übrigen dieser Reste für eine CH₂-Gruppe stehen,
- R¹⁷ und R¹⁸: unabhängig voneinander ein Wasserstoffatom, eine (C₁ bis C₆)-Alkylgruppe, eine Hydroxygruppe, ein Halogenatom oder eine Carboxygruppe bedeuten,
mit der Maßgabe, dass ein Äquivalent eines ein- oder mehrwertigen Anions zugegen ist, wenn M keine negative Ladung bedeutet.

Die CH₂-Gruppe bildet im Ring des Heterozyklus ein Methandiylfragment -CH₂-, das selbstverständlich auch mit einem der Reste R¹⁷ bzw. R¹⁸ substituiert sein kann.

Die zuvor erwähnten bevorzugten Definitionen der Reste M und Y gelten auch hier, allein oder gemeinsam, auf Formeln (la-5) und (la-6) angewendet, als bevorzugt. Die unter der Maßgabe beschriebene Bedingung beschreibt die Ausbildung eines inneren Salzes, das ebenso eine bevorzugte Ausführungsform darstellt.

Wenn der Rest R² der Formel (II) für einen Rest der oben genannten Formel (IV) steht, dann ergibt sich aus der Formel (I) das erfindungsgemäße Sulfinsäurederivat gemäß Formel (la-7), 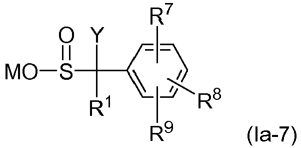 worin M, Y, R¹, R⁷, R⁸ und R⁹ die unter Formel (I) genannten Bedeutungen haben.

Es sind solche Verbindungen der Formel (la-7) erfindungsgemäß bevorzugt, bei welchen der Rest R⁷ eine Carboxygruppe oder eine Sulfonsäuregruppe bedeutet und die Reste R⁸ und R⁹ für ein Wasserstoffatom stehen.

Weiterhin erfindungsgemäß bevorzugt eingesetzte Verbindungen der Formel (I) sind Verbindungen der Formel (la-8) 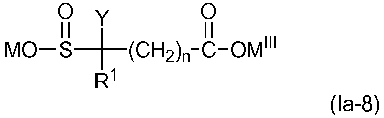 worin M, Y, R¹, n und M^{III} die unter Formel (I) und (II) genannten Bedeutungen haben. Die zuvor erwähnten bevorzugten Definitionen der Reste M, Y, R¹, n, und M^{III} gelten auch hier, allein oder gemeinsam auf Formel (la-8) angewendet, als bevorzugt.

Es sind darüber hinaus solche Verbindungen der Formel (la-8) erfindungsgemäß bevorzugt, bei welchen Y eine Hydroxy- oder Aminogruppe bedeutet.

Bevorzugt steht n in Formel (la-8) für eine Zahl 0, 1 oder 2.

Steht der Rest R der Formel (I) für einen Rest der oben genannten Formel (III), dann ergibt sich aus der Formel (I) als bevorzugte zweite Ausführungsform der Erfindung das erfindungsgemäße Sulfinsäurederivat der Formel (Ib), 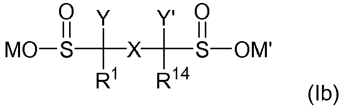 worin M, M', Y, Y', X, R¹ und R¹⁴ die unter den Formeln (I) und (III) definierte Bedeutung haben. Die zuvor erwähnten bevorzugten Definitionen der Reste M, M', Y, Y', X, R¹ und R¹⁴ gelten auch hier, allein oder gemeinsam auf Formel (Ib) angewendet, als bevorzugt.

Der Rest X steht bevorzugt für einen organischen Rest mit zwei freien Valenzen. Als erfindungsgemäße Reste eignen sich prinzipiell alle Organodiyl-Reste, wie z.B. aliphatische oder alizyklische, aromatische oder heteroaromatische Diyl-Reste. Der besagte organische Rest mit zwei freien Valenzen X gemäß Formel (III) bzw. Formel (Ib) wird bevorzugt aus der Gruppe ausgewählt, die gebildet wird aus gegebenenfalls substituierten Resten aus Arendiyl, Heteroarendiyl, Alkandiyl, Alkendiyl, Cycloalkandiyl, Cycloalkendiyl und Di((C₁ bis C₆)alkylen)-substituierten carbozyklischen oder heterozyklischen Gruppen, die aliphatisch oder aromatisch sind. Falls die oben genannten ausgewählten Reste dieser Ausführungsform substituiert sind, dann sind diese bevorzugt mit mindestens einem Rest aus (C₁ bis C₆)-Alkyl, (C₂ bis C₆)-Alkenyl, (C₁ bis C₆)-Hydroxyalkyl, Aryl-(C₁ bis C₆)-alkyl, Hydroxy, (C₁ bis C₆)-Alkoxy, Amino, Di(C₁ bis C₆)alkylamino, Nitro, Halogen, Carbamoyl, Sulfonamido, Cyano oder Carboxamido substituiert.

Es ist erfindungsgemäß besonders bevorzugt, wenn der Rest X der Formel (III) bzw. der Formel (Ib) für einen organischen Rest mit zwei freien Valenzen steht, der aus einer (C₁ bis C₆)-Alkandiylgruppe oder aus Resten der Formeln (VII) bis (XI) ausgewählt wird, 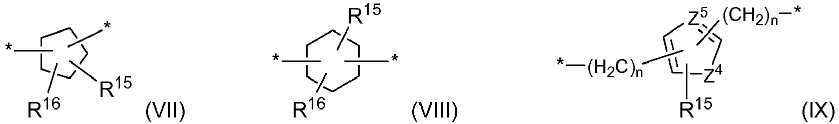 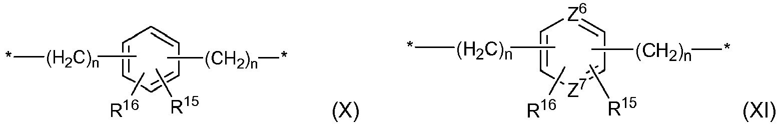 worin bedeuten
- R¹⁵ und R¹⁶: unabhängig voneinander für ein Wasserstoffatom, ein Halogenatom, eine (C₁ bis C₆)-Alkylgruppe oder eine Carboxygruppe,
- n: eine ganze Zahl von 0 bis 6,
- Z⁴: eine Gruppe CH₂, ein Sauerstoffatom, ein Schwefelatom oder eine Gruppe NR', mit R' = Wasserstoffatom, eine (C₁ bis C₆)-Alkylgruppe, eine (C₁ bis C₆)-Hydroxyalkylgruppe oder eine (C₂ bis C₆)-Polyhydroxyalkylgruppe,
- Z⁵, Z⁶ und Z⁷: unabhängig voneinander eine Gruppe CH oder ein Stickstoffatom.

Die jeweiligen Gruppen CH bzw. CH₂ der Reste Z⁴, Z⁵, Z⁶ oder Z⁷ können selbstverständlich erfindungsgemäß ebenso mit allen erfindungsgemäß im Rahmen der Definition der betroffenen Formel aus den Formeln (VII) bis (XI) möglichen Substituenten substituiert sein.

Falls der organische Rest mit zwei freien Valenzen X aus den Formeln (VII) und (VIII) ausgewählt wird, sind die Reste 2-Chlor-cyclopentan-1,3-diyl, 2-Brom-cyclopentan-1,3-diyl, 2-Chlorcyclohexan-1,3-diyl und 2-Brom-cyclohexan-1,3-diyl bevorzugte Vertreter.

Im Folgenden sollen Beispiele für die als Substituenten im Rahmen dieser Anmeldung genannten Gruppen bzw. Reste erwähnt werden. Beispiele für (C₁ bis C₆)-Alkylreste sind lineare, verzweigte oder zyklische (C₁ bis C₆)-Alkylgruppen, wobei lieneare oder verzweigte (C₁ bis C₆)-Alkylgruppen bevorzugt sind. Insbesondere sind die Gruppen Methyl, Ethyl, Propyl, Isopropyl, n-Butyl, sec-Butyl, tert.-Butyl, n-Pentyl und n-Hexyl geeignet. Beispiele für entsprechend geeignete zyklische Alkylgruppen sind Cyclopentyl und Cyclohexyl.
Beispiele für bevorzugte (C₂ bis C₆)-Alkenylreste sind Vinyl, Allyl und Butenyl. Erfindungsgemäß bevorzugte (C₁ bis C₆)-Alkoxyreste sind beispielsweise eine Methoxy- oder eine Ethoxygruppe.
Die Methoxycarbonyl-, Ethoxycarbonyl-, n-Propoxycarbonyl-, Isopropoxycarbonyl-, n-Butoxycarbonyl-, sec-Butoxycarbonyl- und tert-Butoxycarbonylgruppe sind Beispiele für (C₁ bis C₆)-Alkoxycarbonylgruppen; die Methoxycarbonyl- und die Ethoxycarbonylgruppe sind dabei besonders bevorzugt.
Die 2-Methoxycarbonylethyl-, 2-Ethoxycarbonylethyl-, 2-Propoxycarbonylethyl-, 2-Isopropoxycarbonylethyl-, 2-Butoxycarbonylethyl-, 2-sec-Butoxycarbonylethyl-, 2-tert-Butoxycarbonylethyl-, 3-Methoxycarbonylpropyl-, 3-Ethoxycarbonylpropyl-, 3-Propoxycarbonylpropyl-, 3-Isopropoxycarbonylpropyl-, 3-Butoxycarbonylpropyl-, 3-sec-Butoxycarbonylpropyl- und 3-tert-Butoxycarbonylpropylgruppe sind Beispiele für (C₁ bis C₆)-Alkoxycarbonyl-(C₁ bis C₆)-alkylgruppen.
Beispiele für erfindungsgemäße Cyano-(C₁ bis C₆)-alkylgruppen sind Cyanomethyl, 2-Cyanoethyl, 3-Cyanopropyl, 4-Cyanobutyl und 5-Cyanopentyl.
Weiterhin können als bevorzugte Beispiele für eine (C₁ bis C₆)-Monohydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe genannt werden. Eine 2-Hydroxyethylgruppe ist besonders bevorzugt. Beispiele für eine (C₂ bis C₆)-Polyhydroxyalkylgruppe sind die 2,3-Dihydroxypropylgruppe, 3,4-Dihydroxybutylgruppe und die 2,4-Dihydroxybutylgruppe. Die Methoxyethyl-, Ethoxyethyl-, Methoxypropyl-, Methoxybutyl-, Ethoxybutyl- und die Methoxyhexylgruppe sind Beispiele für erfindungsgemäße (C₁ bis C₆)-Alkoxy-(C₁ bis C₆)-alkylgruppen.
Eine bevorzugte Hydroxy-(C₁ bis C₆)-alkoxygruppe ist die 2-Hydroxyethoxygruppe. Bevorzugte Arylgruppen sind Phenyl, Naphthyl und Biphenyl.
Bevorzugte Heteroarylgruppen sind Thienyl, Furyl, Pyrrolyl, Imidazolyl, Thiazolyl, Oxazolyl, Pyrazolyl, Pyridyl, Pyrimidinyl, Pyrazyl, Pyridazyl, Benzimidazolyl, Benzothiazolyl, Benzoxazolyl, Indolyl, Chinolinyl, Chinoxalinyl und Chinazolinyl.
Die Trifluomethyl- und die Pentafluorethylgruppe sind bevorzugte Perfluor-(C₁ bis C₆)-alkylgruppen.
Beispiele für Halogenatome sind F-, Cl-, Br- oder I-Atome, wobei Cl- und Br-Atome ganz besonders bevorzugt sind.
Bevorzugte Aryl-(C₁ bis C₆)-alkylgruppen sind Benzyl und 2-Phenylethyl. Die Trimethylammonium- und Diethylmethylammonium- sind Beispiele für eine Gruppe - N⁺R^{I}R^{II}R^{III}.
Die 2-Trimethylammoniumethyl- ist ein Beispiel für eine N,N,N-Tri[(C₁ bis C₆)-akyl]ammonium-(C₁ bis C₆)-alkylgruppe.
Eine bevorzugte (C₁ bis C₆)-Carboxyalkylgruppe ist die 3-Carboxypropylgruppe. Die weiteren verwendeten Begriffe leiten sich erfindungsgemäß von den hier gegebenen Definitionen ab.

Ganz besonders bevorzugte Vertreter der Sulfinsäurederivate gemäß Formel (I) sind die Sulfinsäuren gemäß folgender Liste oder deren Salze mit einem Äquivalent mindestens eines ein- oder mehrwertigen Kations:

| Name: | korrespondierende Struktur: |
|---|---|
| 2-Furyl(amino)methansulfinsäure, | 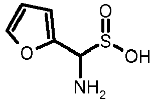 |
| Amino(thien-2-yl)methansulfinsäure, | 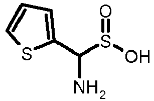 |
| Amino(1*H*-imidazol-2-yl)methansulfinsäure, | 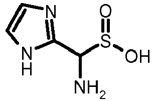 |
| Amino(1,3-thiazol-2-yl)methansulfinsäure, | 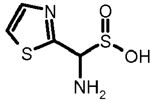 |
| Amino(1,3-oxazol-2-yl)methansulfinsäure, | 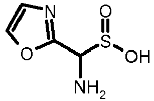 |
| Amino(1*H*-pyrrol-2-yl)methansulfinsäure, | 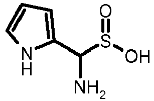 |
| Amino(hydroxyl-2-yl)methansulfinsäure, | 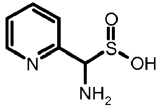 |
| Amino(hydroxyl-4-yl)methansulfinsäure, | 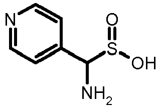 |
| Amino[3-hydroxy-5-(hydroxymethyl)-2-methylpyridin-4-yl]methansulfinsäure, | 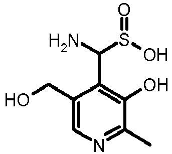 |
| Amino(chinolin-2-yl)methansulfinsäure, | 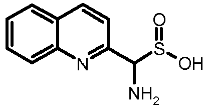 |
| Amino(chinolin-4-yl)methansulfinsäure, | 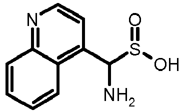 |
| 1*H*-Benzimidazol-2-yl(amino)methansulfinsäure, | 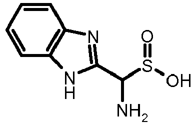 |
| 1,3-Benzothiazol-2-yl(amino)methansulfinsäure, | 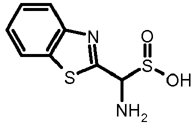 |
| 1,3-Benzoxazol-2-yl(amino)methansulfinsäure, | 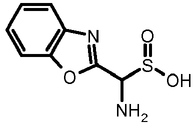 |
| Hydroxy(thien-2-yl)methansulfinsäure, | 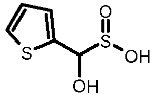 |
| Hydroxy(thien-3-yl)methansulfinsäure | 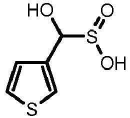 |
| (3-Bromthien-2-yl)(hydroxyl)methansulfinsäure, | 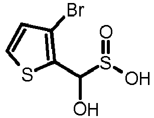 |
| Hydroxy(1H-imidazol-2-yl)methansulfinsäure, | 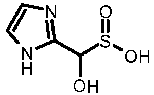 |
| Hydroxy(1H-imidazol-5-yl)methansulfinsäure, | 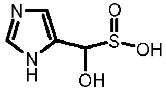 |
| Hydroxy(1-methyl-5-nitro-1*H*-imidazol-2-yl)methansulfinsäure, | 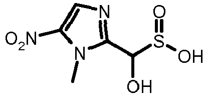 |
| Hydroxy(1,3-thiazol-2-yl)methansulfinsäure, | 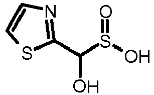 |
| Hydroxy(1,3-oxazol-2-yl)methansulfinsäure, | 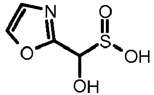 |
| Hydroxy(1*H*-pyrrol-2-yl)methansulfinsäure, | 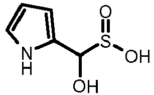 |
| Hydroxy(hydroxyl-2-yl)methansulfinsäure, | 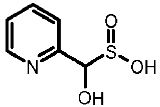 |
| Hydroxy(hydroxyl-4-yl)methansulfinsäure, | 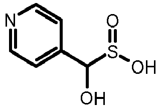 |
| Hydroxy[3-hydroxy-5-(hydroxymethyl)-2-methylpyridin-4-yl]-methansulfinsäure, | 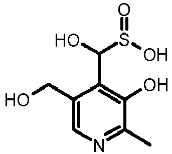 |
| Hydroxy(chinolin-2-yl)methansulfinsäure, | 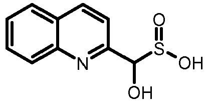 |
| Hydroxy(chinolin-4-yl)methansulfinsäure, | 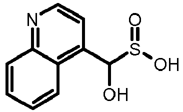 |
| 1*H*-Benzimidazol-2-yl(hydroxyl)methansulfinsäure, | 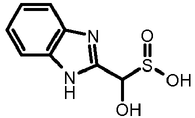 |
| 1,3-Benzothiazol-2-yl(hydroxyl)methansu lfinsäure, | 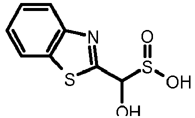 |
| 1,3-Benzoxazol-2-yl(hydroxy)methansulfinsäure, | 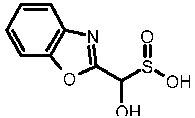 |
| 1,2-Dihydroxyethan-1,2-disulfinsäure, | 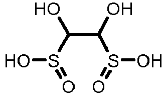 |
| 1,3-Dihydroxypropan-1,3-disulfinsäure, | 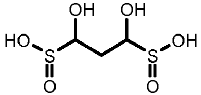 |
| Hydroxy{4-[hydroxy(sulfino)methyl]phenyl}methansulfinsäure, | 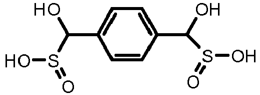 |
| {2-Chlor-3-[hydroxy(sulfino)methyl]cyclohexyl}-(hydroxy)methansu lfinsäure, | 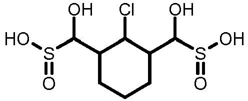 |
| {2-Chlor-3-[hydroxyl(sulfino)methyl]cyclopentyl}-(hydroxyl)methansulfinsäure, | 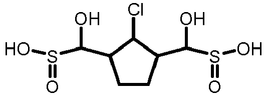 |
| Hydroxy{5-[hydroxy(sulfino)methyl]thien-2-yl}methansulfinsäure, | 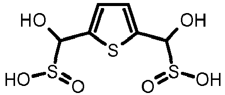 |
| Hydroxy{2-[hydroxy(sulfino)methyl]phenyl}methansulfinsäure, | 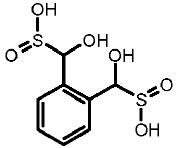 |
| Hydroxy{3-[hydroxy(sulfino)methyl]phenyl}methansulfinsäure, | 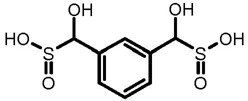 |
| 1,5-Dihydroxypentan-1,5-disulfinsäure, | 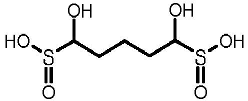 |
| 4-Hydroxy-4-sulfinobutansäure, | 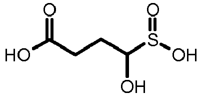 |
| 1-Hydroxy-4-methoxy-4-oxobutan-1-sulfinsäure, | 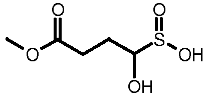 |
| 1-Hydroxy-4-ethoxy-4-oxobutan-1-sulfinsäure, | 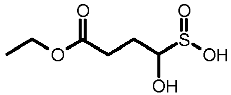 |
| 4-Hydroxy-4-sulfinopentansäure, | 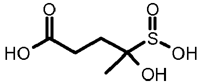 |
| 2-Hydroxy-5-methoxy-5-oxopentan-2-sulfinsäure, | 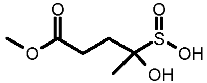 |
| 2-Hydroxy-5-ethoxy-5-oxopentan-2-sulfinsäure, | 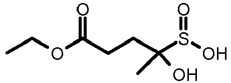 |
| 4-Hydroxy-4-sulfinobut-2-ensäure, | 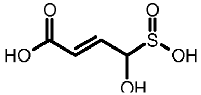 |
| 4-Hydroxy-4-sulfinopent-2-ensäure, | 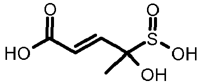 |
| 5-Hydroxy-5-sulfinopentansäure, | 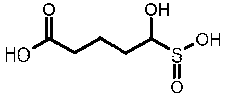 |
| 1-Hydroxy-5-methoxy-5-oxopentan-1-sulfinsäure, | 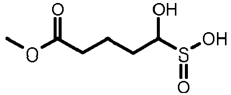 |
| 1-Hydroxy-5-ethoxy-5-oxopentan-1-sulfinsäure, | 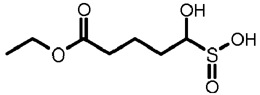 |
| 5-Hydroxy-5-sulfinohexansäure, | 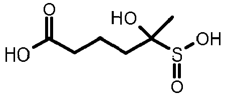 |
| 2-Hydroxy-6-methoxy-6-oxohexan-2-sulfinsäure, | 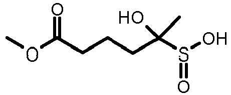 |
| 2-Hydroxy-6-ethoxy-6-oxohexan-2-sulfinsäure, | 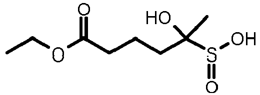 |
| 4-[Hydroxy(sulfino)methyl]benzoesäure, | 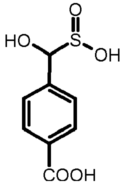 |
| 4-[Amino(sulfino)methyl]benzoesäure, | 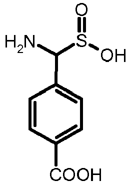 |
| 3-Hydroxy-4-[hydroxy(sulfino)methyl]benzoesäure, | 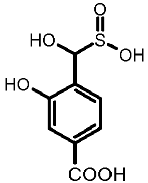 |
| 3-Hydroxy-4-[amino(sulfino)methyl]benzoesäure, | 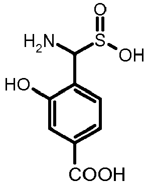 |
| 2-Hydroxy-2-sulfino-essigsäure | 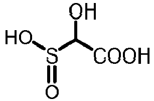 |
| 2-Amino-2-sulfino-essigsäure | 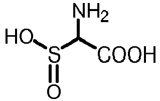 |
| Salz des 4-Hydroxy-1,1-dimethyl-4-sulfinopiperidiniums mit An⁻ als Äquivalent eines ein- oder mehrwertigen Anions, | 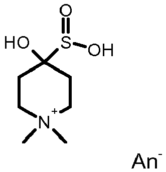 |
| Salz des 1-Allyl-4-hydroxy-1-methyl-4-sulfinopiperidiniums mit An⁻ als Äquivalent eines ein- oder mehrwertigen Anions, | 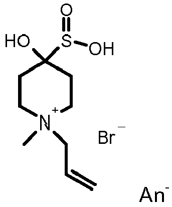 |
| 2-Hydroxy-2-sulfino-propionsäure | 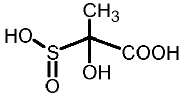 |
| 2-Amino-2-sulfino-propionsäure | 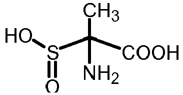 |
| 2-Hydroxy-2-sulfinato-essigsäureethylester | 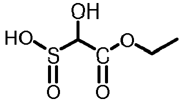 |
| 2-Amino-2-sulfinato-essigsäureethylester | 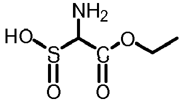 |
| 4-Hydroxy-1,1-dimethylpiperidinium-4-sulfinat, | 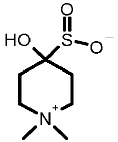 |
| 1-Allyl-4-hydroxy-1-methylpiperidinium-4-sulfinat, | 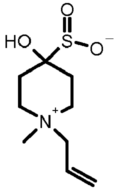 |
| 1-Hydroxy-2-(trimethylammonio)ethansulfinat, | 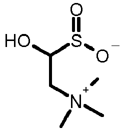 |
| 4-[Hydroxy(sulfino)methyl]benzolsulfonsäure und | 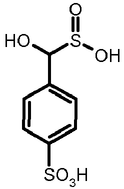 |
| 2-[Hydroxy(sulfino)methyl]benzolsulfonsäure | 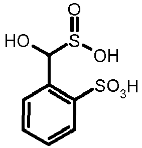 |
| 3-Hydroxy-3-sulfinato-propionsäure | 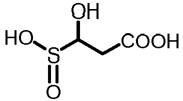 |
| 3-Amino-3-sulfinato-propionsäure | 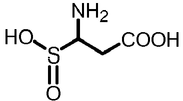 |
| 1-Hydroxy-2-methoxy-2-oxoethansulfinsäure | 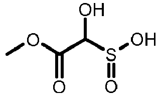 |
| 2-Amino-1-hydroxy-2-oxoethansulfinsäure | 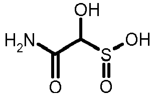 |
| 2-(Dimethylamino)-1-hydroxy-2-oxoethansulfinsäure | 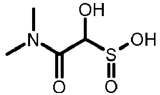 |
| Hydroxy(sulfino)methansulfonsäure | 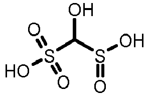 |

Für die bevorzugten ein- oder mehrwertigen Kationen aller Salze der zuvor genannten Verbindungen gilt das zuvor Gesagte.

Die Herstellung der erfindungsgemäß eingesetzten Sulfinsäuren der Formel (I) erfolgt beispielsweise aus der Umsetzung von Dithionit mit entsprechenden Aldehyden bzw. Ketonen analog zu der Herstellvorschrift gemäß WO-A1-99/18067.

Bevorzugt enthält das erfindungsgemäße Mittel mindestens ein Sulfinsäurederivat der Formel (I) in einer Menge von 0,01 bis 20 Gew.-%, besonders bevorzugt von 1 bis 20 Gew.-%, jeweils bezogen auf das Gewicht des Mittels.

Erfindungsgemäß wird mindestens ein spezieller cyclischer, linearer oder verzweigter aliphatischer Aldehyd eingesetzt.

Unter cyclischen, linearen oder verzweigten aliphatischen Aldehyden sind erfindungsgemäß aliphatische Aldehyde zu verstehen, deren Formylgruppe(n) nicht in Konjugation mit einem aromatischen π-Elektronen-System stehen. Die entsprechenden Aldehyde dürfen aromatische Reste tragen, solange die π-Etektronen der Formylgruppe(n) nicht über ein solches aromatisches System delokalisiert sein können. Erfindungsgemäße cyclische, lineare oder verzweigte aliphatische Aldehyde sind gesättigt oder ungesättigt und werden ausgewählt aus mindestens einem Vertreter aus der Gruppe Formaldehyd, Acetaldehyd, Glyoxal, Propionaldehyd, Butanal, Pentanal, Isopentanal, Hexanal, Cyclohexanal, Heptanal, Octanal, Malondialdehyd, Glutaraldehyd, 2-Methylpentanal, 2-Ethylhexanal, 3,5,5-Trimethylhexanal, 2-Ethylbutyraldehyd, 2-Methylbutyraldehyd, Isobutyraldehyd, 3-Phenylpropanal, 3-(4-Methylphenyl)propanal, 3-(4-Methoxyphenyl)propanal, 3-(2-Methoxyphenyl)propanal, 2-Butenal, Acrolein, 3-Methyl-2-butenal, 3,7-Dimethyl-2,6-octadienal, 2,4-Pentadienal, 3,7-Dimethyl-6-octenal, 2,4-Dimethyl-3-cyclohexencarboxaldehyd und 2,6-Nonadienal.

Die Verbindungen, ausgewählt aus Aldehyden oder Ketonen, sind erfindungsgemäß bevorzugt in einer Menge von 0,1 Gew.-% bis 20 Gew.-%, insbesondere von 0,5 Gew.-% bis 10 Gew.-%, jeweils bezogen auf das Gewicht des anwendungsbereiten Mittels, in dem erfindungsgemäßen Mittel enthalten.

Die erfindungsgemäßen Mittel werden im Allgemeinen auf die Weise hergestellt, dass mindestens ein Sulfinsäurederivat der Formel (I) und mindestens eine Verbindung, ausgewählt aus den Aldehyden und/oder den Ketonen, unter Rühren dem Träger beigemischt werden. Dabei ist es unwesentlich, in welcher Reihenfolge die beiden Komponenten dem vorgelegten Träger zugemischt werden. Ebenso können mindestens ein Sulfinsäurederivat der Formel (I) und mindestens eine Verbindung, ausgewählt aus den zuvor angeführten speziellen Aldehyden, nacheinander in einem Mischgefäß vorgelegt und unter Rühren der Träger hinzugefügt werden.
Es kann erfindungsgemäß bevorzugt sein, das erfindungsgemäße Mittel mit der Maßgabe bereitzustellen, dass die in dem erfindungsgemäßen Mittel eingesetzten Verbindungen, ausgewählt aus den zuvor angeführten speziellen Aldehyden, sich von denjenigen Aldehyden bzw. Ketonen unterscheiden müssen, von denen sich diejenigen Sulfinsäuren ableiten, die in dem erfindungsgemäßen Mittel tatsächlich enthalten sind.

Als Träger für das erfindungsgemäße Mittel eignen sich bevorzugt flüssige Medien, in denen das erfindungsgemäße Sulfinsäurederivat bevorzugt löslich ist, wie beispielsweise Wasser oder organische Lösemittel. Es ist erfindungsgemäß bevorzugt, wenn der Träger ein kosmetischer Träger ist.

Als kosmetische Träger eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige
Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.
Unter wässrig-alkoholischen kosmetischen Trägern sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Weitere alkoholische Lösemittel, sind beispielsweise Methoxybutanol, Benzylalkohol, 2-Phenoxyethanol, Ethyldiglykol oder 1,2-Propylenglykol. In einer bevorzugten Ausführungsform enthält das erfindungsgemäße Mittel zusätzlich als Lösemittel mindestens einen (C₂ bis C₆)-Alkylmonoalkohol und/oder ein (C₂ bis C₆)-Alkandiol, insbesondere Ethanol, Isopropanol und/oder 1,2-Propylenglykol.

Das erfindungsgemäße Mittel besitzt bevorzugt einen pH-Wert von pH 1 bis pH 9, insbesondere von pH 1,5 bis pH 6.

Ein zweiter Gegenstand der Erfindung ist die Verwendung des Mittels gemäß erstem Erfindungsgegenstand zur Entfärbung von Substraten, die mit natürlichen und/oder synthetischen Farbstoffen eingefärbt sind.

Das Substrat enthält natürliche Fasern.
Die natürlichen Fasern werden ausgewählt aus keratinhaltigen Fasern, insbesondere Wolle oder tierischen oder menschlichen Haaren, ganz besonders bevorzugt aus menschlichen Haaren.

Die zu entfärbenden Substrate sind bevorzugt mit Oxidationsfarbstoffen und/oder direktziehenden Farbstoffen, als Vertreter der synthetischen Farbstoffe, gefärbt.
In einer weiteren bevorzugten Ausführungsform wurden die zu entfärbenden Substrate mit einem oxidativen Färbemittel gefärbt, welches neben mindestens einer Entwicklerkomponente zusätzlich mindestens eine Kupplerkomponente enthält.
Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlorresorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.
Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Zusätzlich können die zu entfärbenden Substrate mit Vorstufen naturanaloger Farbstoffe bevorzugt unter Zuhilfenahme solcher Indole und Indoline gefärbt sein, die bevorzugt mindestens zwei Hydroxy- oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.
Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin. Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.
Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.
Das zu entfärbende Substrat kann ebenso mit direktziehenden Farbstoffen eingefärbt worden sein. Als direktziehende Farbstoffe kommen dabei insbesondere Nitrophenylendiamine, Nitroaminophenole, Azofarbstoffe, Anthrachinone oder Indophenole in Frage. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.
Ferner können die zu entfärbenden Substrate bevorzugt mit einem kationischen direktziehenden Farbstoff gefärbt sein. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Ein dritter Gegenstand der Erfindung ist ein Verfahren zur reduktiven Entfärbung von mit natürlichen und/oder synthetischen Farbstoffen eingefärbten Substraten, in dem ein Mittel des ersten Erfindungsgegenstandes auf das eingefärbte Substrat aufgetragen wird und nach einer Einwirkzeit wieder abgespült wird.

Die Einwirkzeit beträgt bevorzugt 1 bis 60 Minuten, bevorzugt 5 bis 30 Minuten. Die Einwirkung des erfindungsgemäßen Mittels kann nicht nur bei Raumtemperatur, sondern bevorzugt in einem Temperaturbereich von 15 bis 60 °C, insbesondere von 25 bis 60 °C erfolgen.
Nach Ablauf der Einwirkzeit werden die Haare ausgespült, wobei bevorzugt ein tensidhaltiges Mittel, wie beispielsweise ein Reinigungsmittel oder ein Shampoo, Anwendung findet. Gegebenenfalls kann das Substrat mehrfach ausgespült, bzw. mit dem tensidhaltigen Mittel behandelt werden.
Nach dem Ausspülen kann es vorteilhaft sein, das Substrat mit einer Oxidationsmittelhaltigen Zusammensetzung zu behandeln. Bevorzugt wird Wasserstoffperoxid als Oxidationsmittel, bevorzugt in Konzentrationen von 0,5 bis 6 Gew.-%, eingesetzt. Die Einwirkzeit beträgt bevorzugt 1 bis 30 Minuten, besonders bevorzugt 1 bis 10 Minuten. Nach Ablauf der Einwirkzeit wird die Oxidationsmittelhaltige Zusammensetzug ausgespült.

Die eingefärbten Substrate sind dieselben, die im zweiten Erfindungsgegenstand definiert wurden.

Als bevorzugte natürliche oder synthetische Farbstoffe wurden bevorzugt diejenigen Farbstoffe verwendet, wir sie im zweiten Erfindungsgegenstand definiert wurden.

Der Erfindungsgegenstand soll exemplarisch anhand der folgenden Ausführungen erläutert werden.

### Beispiele

Es wurden die Entfärbemittel gemäß Tabelle 1 bereitgestellt. Dabei wurden folgende Rohstoffe verwendet:
Brüggolit FF7 der Fa. Brüggemann: enthält ca. 65 % 2-Hydroxy-2-sulfinoessigsäure Natriumsalz

Die Rohstoffe wurden nach und nach in 75 Gew.-% Wasser unter Rühren gemischt. Anschließend wurde der pH-Wert eingestellt und ggf. mit Wasser auf 100 Gew.-% aufgefüllt.

**Tabelle 1: Entfärbemittel**

| Rohstoff | E1** | E2* | E3** |
|---|---|---|---|
| | [Gew.-%] | [Gew.-%] | [Gew.-%] |
| Brüggolit FF7 | 10,0 | 10,0 | 10,0 |
| Benzaldehyd | 2,0 | - | - |
| Glyoxal | - | 2,0 | - |
| Allantoin | - | - | 2,0 |
| Natriumlaurethsulfat | 2,0 | - | - |
| H₃PO₄ | ad pH 1,5 | ad pH 1,5 | ad pH 1,5 |
| Wasser | ad 100 | ad 100 | ad 100 |

| | | | |
|---|---|---|---|
| *erfindungsgemäß **nicht erfindungsgemäß | | | |

Mit handelsüblichen oxidativen Haarfarben gefärbte Haarsträhnen wurden jeweils mit einem der Entfärbemittel der Tabelle 1 (Flottenverhältnis 4 g Entfärbemittel auf 1 g Haarsträhne) über eine Einwirkzeit von 30 Minuten bei Raumtemperatur behandelt und anschließend mit Wasser gespült.

Alle Haarsträhnen zeigten nach der Entfärbeprozedur eine hervorragende Aufhellung und eine geringe Haarschädigung. Die Haarsträhnen erfuhren auch nach 2 Wochen Lagerung keine Nachdunkelung.

## Patentansprüche

1. Mittel zum reduktiven Farbabzug keratinhaltiger Fasern, enthaltend in einem Träger mindestens ein Sulfinsäurederivat der Formel (I) 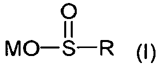 worin
M steht für ein Wasserstoffatom oder ein Äquivalent eines ein- oder mehrwertigen Kations,
R für einen Rest gemäß einer der Formeln (II) bis (III) steht, 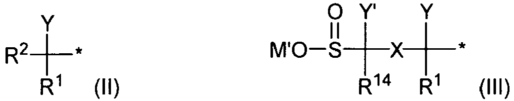 worin bedeuten
Y und Y' unabhängig voneinander eine Hydroxygruppe, eine -NH₂ Gruppe oder eine Gruppe -NR³R⁴, wobei R³ und R⁴ unabhängig voneinander für eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine Arylgruppe oder eine Aryl-(C₁ bis C₆)-alkylgruppe stehen,
M' unabhängig von M die unter M aufgeführten Merkmale,
X eine direkte Bindung oder einen organischen Rest mit zwei freien Valenzen,
R¹ und R¹⁴ unabhängig voneinander ein Wasserstoffatom, eine (C₁ bis C₆)-Alkylgruppe oder eine Carboxyalkylgruppe -(CH₂)ₘ-COOM^{II}, in der M^{II} für ein Wasserstoffatom oder für ein Äquivalent eines ein- oder mehrwertigen Kations steht und m die Zahl 0, 1 oder 2 bedeutet,
R² ein Wasserstoffatom, eine (C₁ bis C₆)-Alkylgruppe, einen Carboxyalkylrest -(CH₂)ₙ-COOM^{III} mit
M^{III} = Wasserstoffatom oder ein Äquivalent eines ein- oder mehrwertigen Kations und n eine ganze Zahl 0, 1, 2, 3, 4, 5 oder 6,
einen (C₁ bis C₆)-Alkyloxycarbonylrest, eine Sulfonsäuregruppe, eine Carbamoylgruppe, eine N,N-Di[(C₁ bis C₆)-alkyl]carbamoylgruppe, eine N,N,N-Tri[(C₁ bis C₆)-alkyl]ammonium-(C₁ bis C₆)-alkylgruppe eine Carboxy-(C₂ bis C₆)-alkenylgruppe, eine Cyano-(Ci bis C₆)-alkylgruppe oder eine (C₁ bis C₆)-Alkoxycarbonyl-(C₁ bis C₆)-alkylgruppe, oder
R² zusammen mit R¹ und dem Restmolekül einen aliphatischen 5-, 6-, oder 7-gliedrigen Ring bildet, welcher mindestens ein kationisches, quaterniertes Stickstoffatom als Heteroatom enthält, wobei die kationische Ladung gegebenenfalls durch ein Äquivalent eines ein- oder mehrwertigen Anions kompensiert wird, oder
einen aliphatischen oder aromatischen Heterozyklus, der substituiert sein kann, oder
einen Rest gemäß Formel (IV) 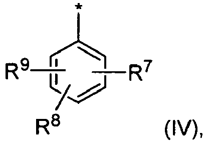 worin
R⁷ eine Carboxygruppe, eine Sulfonsäuregruppe, eine (C₁ bis C₆)-Alkylgruppe, eine (C₂ bis C₆)-Alkenylgruppe, eine Aryl(C₁ bis C₆)-alkylgruppe oder eine (C₁ bis C₆)-Hydroxyalkylgruppe bedeutet,
R⁸ und R⁹ unabhängig voneinander ein Wasserstoffatom, eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine (C₁ bis C₆)-Alkoxygruppe, eine Hydroxygruppe, eine Aminogruppe, eine Carboxygruppe, eine Nitrogruppe, eine Cyanogruppe oder ein Halogenatom bedeuten,
mit der Maßgabe, dass Wasserstoffatom stehen, in Formel (II) R¹ und R² nicht gleichzeitig für ein
in Kombination mit mindestens einer Verbindung, ausgewählt aus den Aldehyden, wobei die Verbindung aus den Aldehyden ausgewählt wird aus mindestens einem Vertreter aus der Gruppe, die gebildet wird, aus cyclischen, linearen oder verzweigten aliphatischen Aldehyden und wobei die cyclischen, linearen oder verzweigten Aldehyde ausgewählt werden aus mindestens einem Vertreter aus der Gruppe Formaldehyd, Acetaldehyd, Glyoxal, Propionaldehyd, Butanal, Pentanal, Isopentanal, Hexanal, Cyclohexanal, Heptanal, Octanal, Malondialdehyd, Glutaraldehyd, 2-Methylpentanal, 2-Ethylhexanal, 3,5,5-Trimethylhexanal, 2-Ethylbutyraldehyd, 2-Methylbutyraldehyd, Isobutyraldehyd, 3-Phenylpropanal, 3-(4-Methylphenyl)propanal, 3-(4-Methoxyphenyl)propanal, 3-(2-Methoxyphenyl)propanal, 2-Butenal, Acrolein, 3-Methyl-2-butenal, 3,7-Dimethyl-2,6-octadienal, 2,4-Pentadienal, 3,7-Dimethyl-6-octenal, 2,4-Dimethyl-3-cyclohexencarboxaldehyd und 2,6-Nonadienal.

2. Mittel nach Anspruch 1, **dadurch gekennzeichnet, daß** gemäß Formel (I) die Reste Y der Formel (II) bzw. der Formel (III) und Y' der Formel (III) unabhängig voneinander eine Hydroxygruppe oder eine Gruppe -NH₂ bedeuten.

3. Mittel nach einem der Ansprüche 1 oder 2, **dadurch gekennzeichnet, daß** es das Sulfinsäurederivat in einer Menge von 0,01 bis 20 Gew.-%, besonders bevorzugt von 1 bis 20 Gew.-%, jeweils bezogen auf das Gewicht des Mittels, enthält.

4. Mittel nach einem der Ansprüche 1 bis 3, **dadurch gekennzeichnet, daß** die Verbindungen, ausgewählt aus Aldehyden oder Ketonen in einer Menge von 0,1 Gew.-% bis 20 Gew.-%, insbesondere von 0,5 Gew.-% bis 10 Gew.-%, jeweils bezogen auf das Gewicht des anwendungsbereiten Mittels, enthalten sind.

5. Mittel nach einem der Ansprüche 1 bis 4, **dadurch gekennzeichnet, daß** es einen pH-Wert von pH 1 bis pH 9, insbesondere von pH 1,5 bis pH 6, besitzt.

6. Verwendung eines Mittels gemäß einem der Ansprüche 1 bis 5 zur Entfärbung von Substraten, die mit natürlichen und/oder synthetischen Farbstoffen eingefärbt sind, wobei die Substrate natürliche Fasern, ausgewählt aus keratinhaltigen Fasern, enthalten.

7. Verfahren zur reduktiven Entfärbung von mit natürlichen und/oder synthetischen Farbstoffen eingefärbten Substraten, wobei die Substrate natürliche Fasern, ausgewählt aus keratinhaltigen Fasern, enthalten, **dadurch gekennzeichnet, daß** ein Mittel gemäß einem der Ansprüche 1 bis 5 auf das eingefärbte Substrat aufgetragen wird und nach einer Einwirkzeit wieder abgespült wird.

## Claims

1. An agent for reductively removing color from keratin-containing fibers, containing in a carrier at least one sulfinic acid derivative of formula (I) 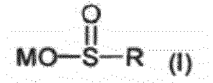 in which
M represents a hydrogen atom or an equivalent of a monovalent or polyvalent cation,
R represents a functional group according to one of formulae (II) to (III), 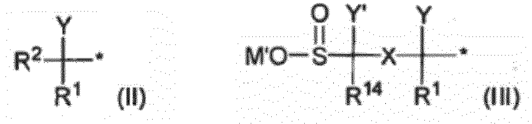
in which
Y and Y' denote, independently of one another, a hydroxy group, an -NH₂ group or an -NR³R⁴ group, wherein R³ and R⁴ represent, independently of one another, a (C₁ to C₆) alkyl group, a (C₂ to C₆) alkenyl group, a (C₁ to C₆) hydroxyalkyl group, a (C₂ to C₆) polyhydroxyalkyl group, an aryl group or an aryl-(C₁ to C₆) alkyl group,
M' denotes, independently of M, the features listed under M,
X denotes a direct bond or an organic functional group having two free valencies,
R¹ und R¹⁴ denote, independently of one another, a hydrogen atom, a (C₁ to C₆) alkyl group or a carboxyalkyl group -(CH₂)ₘ-COOM^{II}, in which M" represents a hydrogen atom or an equivalent of a monovalent or polyvalent cation and m denotes the number 0, 1 or 2,
R² denotes a hydrogen atom, a (C₁ to C₆) alkyl group, a carboxyalkyl functional group-(CH₂)ₙ-COOM^{III} where M^{III} = hydrogen atom or an equivalent of a monovalent or polyvalent cation and n denotes an integer 0, 1, 2, 3, 4, 5 or 6, a (C₁ to C₆) alkyloxycarbonyl functional group, a sulfonic acid group, a carbamoyl group, an N,N-di[(C₁ to C₆) alkyl] carbamoyl group, an N,N,N-tri[(C₁ to C₆) alkyl]ammonium-(C₁ to C₆) alkyl group, a carboxy-(C₂ to C₆) alkenyl group, a cyano-(Ci to C₆) alkyl group or a (C₁ to C₆) alkoxycarbonyl-(C₁ to C₆) alkyl group, or R², together with R¹ and the remainder of the molecule, denotes an aliphatic 5-, 6- or 7-membered ring, which contains at least one cationic, quaternized nitrogen atom as the heteroatom, wherein the cationic charge is optionally compensated for by an equivalent of a monovalent or polyvalent anion, or an aliphatic or aromatic heterocycle, which may be substituted, or a functional group according to formula (IV) 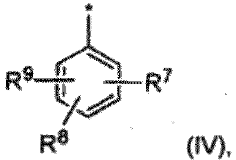
in which
R⁷ denotes a carboxy group, a sulfonic acid group, a (C₁ to C₆) alkyl group, a (C₂ to C₆) alkenyl group, an aryl-(C₁ to C₆) alkyl group or a (C₁ to C₆) hydroxyalkyl group,
R⁸ and R⁹ denote, independently of one another, a hydrogen atom, a (C₁ to C₆) alkyl group, a (C₂ to C₆) alkenyl group, a (C₁ to C₆) hydroxyalkyl group, a (C₂ to C₆) polyhydroxyalkyl group, a (C₁ to C₆) alkoxy group, a hydroxy group, an amino group, a carboxy group, a nitro group, a cyano group or a halogen atom,
with the proviso that, in formula (II), R¹ and R² do not simultaneously represent a hydrogen atom, in combination with at least one compound, selected from the aldehydes, wherein the compound from the aldehydes is selected from at least one representative from the group formed of cyclic, linear or branched aliphatic aldehydes and wherein the cyclic, linear or branched aldehydes are selected from at least one representative from the group formaldehyde, acetaldehyde, glyoxal, propionaldehyde, butanal, pentanal, isopentanal, hexanal, cyclohexanal, heptanal, octanal, malondialdehyde, glutaraldehyde, 2-methylpentanal, 2-ethylhexanal, 3,5,5-trimethylhexanal, 2-ethylbutyraldehyde, 2-methylbutyraldehyde, isobutyraldehyde, 3-phenylpropanal, 3-(4-methylphenyl)propanal, 3-(4-methoxyphenyl)propanal, 3-(2-methoxyphenyl)propanal, 2-butenal, acrolein, 3-methyl-2-butenal, 3,7-dimethyl-2,6-octadienal, 2,4-pentadienal, 3,7-dimethyl-6-octenal, 2,4-dimethyl-3-cyclohexenecarboxaldehyde and 2,6-nonadienal.

2. The agent according to claim 1, **characterized in that**, according to formula (I), the functional groups of formula (II) or formula (III) and Y' of formula (III) denote, independently of one another, a hydroxy group or an -NH₂ group.

3. The agent according to one of claims 1 or 2, **characterized in that** it contains the sulfinic acid derivative in an amount of from 0.01 to 20 wt.%, particularly preferably from 1 to 20 wt.%, in each case based on the weight of the agent.

4. The agent according to one of claims 1 to 3, **characterized in that** the compounds, selected from aldehydes or ketones, are contained in an amount of from 0.1 wt.% to 20 wt.%, in particular from 0.5 wt.% to 10 wt.%, in each case based on the weight of the ready-to-apply agent.

5. The agent according to one of claims 1 to 4, **characterized in that** it has a pH of from 1 to 9, in particular of from 1.5 to 6.

6. The use of an agent according to one of claims 1 to 5 for decolorizing substrates that are colored with natural and/or synthetic dyes, wherein the substrates contain natural fibers, selected from keratin-containing fibers.

7. A method for reductively decolorizing substrates colored with natural and/or synthetic dyes, the substrates containing natural fibers, selected from keratin-containing fibers, **characterized in that** an agent according to one of claims 1 to 5 is applied to the colored substrate and rinsed off again after a contact time.

## Revendications

1. Agent de décoloration réductive de fibres contenant de la kératine, comprenant, dans un support, au moins un dérivé d'acide sulfinique de formule (I) 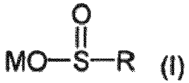 dans laquelle
M représente un atome d'hydrogène ou un équivalent d'un cation mono- ou polyvalent,
R représente un radical selon l'une des formules (II) à (III), 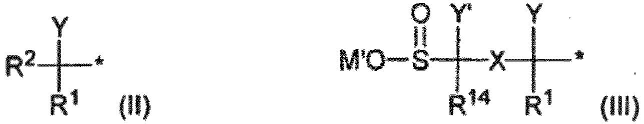
dans lesquelles :
Y et Y' renvoient, indépendamment l'un de l'autre, à un groupe hydroxy, à un groupe -NH₂ ou à un groupe -NR³R⁴, R³ et R⁴ représentant, indépendamment l'un de l'autre, un groupe alkyle en (C₁ à C₆), un groupe alcényle en (C₂ à C₆), un groupe hydroxyalkyle en (C₁ à C₆), un groupe polyhydroxyalkyle en (C₂ à C₆), un groupe aryle ou un groupe aryl(C₁ à C₆)-alkyle,
M' renvoie, indépendamment de M, aux caractéristiques énumérées sous M,
X renvoie à une liaison directe ou à un radical organique ayant deux valences libres,
R¹ et R¹⁴ renvoient, indépendamment l'un de l'autre, à un atome d'hydrogène, à un groupe alkyle en (C₁ à C₆) ou à un groupe carboxyalkyle -(CH₂)ₘ-COOM^{II}, dans lequel M^{II} représente un atome d'hydrogène ou un équivalent d'un cation mono- ou polyvalent, et m, le chiffre 0, 1 ou 2,
R² renvoie à un atome d'hydrogène, à un groupe alkyle en (C₁ à C₆), à un radical carboxyalkyle -(CH₂)ₙ-COOM^{III} où M^{III} = un atome d'hydrogène ou un équivalent d'un cation mono- ou polyvalent, et n, un entier égal à 0, 1, 2, 3, 4, 5 ou 6,
à un radical alkyloxycarbonyle en (C₁ à C₆), à un groupe acide sulfonique, à un groupe carbamoyle, à un groupe carbamoyle N,N-di[alkyl en (C₁ à C₆)], à un groupe alkyle N,N,N-Tri[(C₁ à C₆)-alkyl]ammonium-(C₁ à C₆), à un groupe alcényle carboxy en (C₂ à C₆), à un groupe alkyle cyano en (C₁ à C₆) ou à un groupe alkyle en (C₁ à C₆)-alkoxycarbonyl-(C₁ à C₆), ou
R², avec R¹ et le reste de la molécule, forme un cycle aliphatique à 5, 6 ou 7 chaînons, lequel contient au moins un atome d'azote cationique quaternisé en tant qu'hétéroatome, la charge cationique étant éventuellement compensée par un équivalent d'un anion mono- ou polyvalent, ou
un hétérocycle aliphatique ou aromatique pouvant être substitué, ou un radical selon la formule (IV) 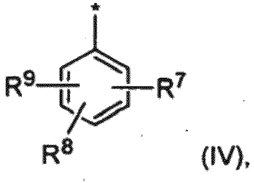 dans laquelle
R⁷ représente un groupe carboxy, un groupe acide sulfonique, un groupe alkyle en (C₁ à C₆), un groupe alcényle en (C₂ à C₆), un groupe aryl(C₁ à C₆)-alkyle ou un groupe hydroxyalkyle en (C₁ à C₆),
R⁸ et R⁹ représentent, indépendamment l'un de l'autre, un atome d'hydrogène, un groupe alkyle en (C₁ à C₆), un groupe alcényle en (C₂ à C₆), un groupe hydroxyalkyle en (C₁ à C₆), un groupe polyhydroxyalkyle en (C₂ à C₆), un groupe alcoxy en (C₁ à C₆), un groupe hydroxy, un groupe amino, un groupe carboxy, un groupe nitro, un groupe cyano ou un atome halogène,
à condition que, dans la formule (II), R¹ et R² ne représentent pas simultanément un atome d'hydrogène,
en combinaison avec au moins un composé choisi parmi les aldéhydes, le composé des aldéhydes étant choisi parmi au moins un représentant du groupe constitué des aldéhydes aliphatiques cycliques, linéaires ou ramifiés, et les aldéhydes cycliques, linéaires ou ramifiés étant choisis parmi au moins un représentant du groupe formaldéhyde, acétaldéhyde, glyoxal, propionaldéhyde, butanal, pentanal, isopentanal, hexanal, cyclohexanal, heptanal, octanal, malondialdéhyde, glutaraldéhyde, 2-méthylpentanal, 2-éthylhexanal, 3,5,5-diméthylhexanal, 2-éthylbutyraldéhyde, 2-méthylbutyraldéhyde, isobutyraldéhyde, 3-phénylpropanal, 3-(4-méthylphényl)propanal, 3-(4-méthoxyphényl)propanal, 3-(2-méthoxyphényl)propanal, 2-buténal, acroléine, 3-méthyl-2-buténal, 3,7-diméthyl-2,6-octadiénal, 2,4-pentadiénal, 3,7-diméthyl-6-octénal, 2,4-diméthyl-3-cyclohexènecarboxaldéhyde et 2,6-nonadiénal.

2. Agent selon la revendication 1, **caractérisé en ce que**, selon la formule (I), les radicaux Y de formule (II) ou de formule (III) et Y' de formule (III) représentent, indépendamment l'un de l'autre, un groupe hydroxy ou un groupe -NH₂.

3. Agent selon l'une des revendications 1 ou 2, **caractérisé en ce qu'**il contient le dérivé d'acide sulfinique en une quantité de 0,01 à 20 % en poids, de manière particulièrement préférée de 1 à 20 % en poids, à chaque fois par rapport au poids de l'agent.

4. Agent selon l'une des revendications 1 à 3, **caractérisé en ce que** les composés, choisis parmi les aldéhydes ou les cétones en une quantité de 0,1 % en poids à 20 % en poids, en particulier de 0,5 % en poids à 10 % en poids, à chaque fois par rapport au poids de l'agent prêt à l'emploi, sont inclus.

5. Agent selon l'une des revendications 1 à 4, **caractérisé en ce qu'**il présente un pH de 1 à 9, en particulier de 1,5 à 6.

6. Utilisation d'un agent selon l'une quelconque des revendications 1 à 5 pour la décoloration de substrats colorés avec des colorants naturels et/ou synthétiques, dans laquelle les substrats contiennent des fibres naturelles choisies parmi les fibres contenant de la kératine.

7. Procédé de décoloration réductive de substrats colorés avec des colorants naturels et/ou synthétiques, dans lequel les substrats contiennent des fibres naturelles choisies parmi les fibres contenant de la kératine, **caractérisé en ce qu'**un agent selon l'une des revendications 1 à 5 est appliqué sur le substrat coloré et rincé à nouveau après un temps de pose.
